# EUROPEAN PATENT SPECIFICATION

(11) **EP 1 849 256 B1**
(45) Date of publication and mention of the grant of the patent: **21.04.2010**
(21) Application number: 05708019.4
(22) Date of filing: 15.02.2005
(51) Int. Cl.: H04L 12/18

(54) **METHOD AND APPARATUS FOR MANAGING MULTICAST TRANSMISSION COSTS**
VERFAHREN UND VORRICHTUNG ZUR VERWALTUNG VON MULTICAST-ÜBERTRAGUNGSKOSTEN
PROCEDE ET DISPOSITIF POUR LA GESTION DE COUTS DE TRANSMISSION MULTIDIFFUSION

(43) Date of publication of application: 31.10.2007
(60) Divisional application: 08164962.6
(73) Proprietor: NTT DoCoMo, Inc., Tokyo (JP)
(72) Inventor: MENDES, Paulo, 81675 München (DE)
(74) Representative: Betten & Resch
(86) International application number: PCT/EP2005/050662
(87) International publication number: WO 2006/087033

(56) References cited:
- EP-A- 1 341 341
- WO-A-02/091670
- US-B- 6 778 553

## Description

### FIELD OF THE INVENTION

The present invention relates to multicast transmission, in particular to the management of multicast transmission costs.

### BACKGROUND ART

Multicast transmission or multicast routing is a method to achieve an efficient network usage. In multicast routing there is created a tree-like path which connects a source and several receivers, the tree containing nodes where it branches such that starting from the source there is a transmission path to each of the receivers. Due to the tree structure the transmission paths are partly identical for different receivers up to a node in the tree where the path branches. The particular network efficiency is achieved by sending only one copy of the packet over each link of the tree. In each branch point the packet is duplicated and a copy of the packet is sent over each downstream link. "Downstream" here refers to the direction from the source to the receiver, whereas in the following "upstream" is referring to the direction from the receiver to the source.

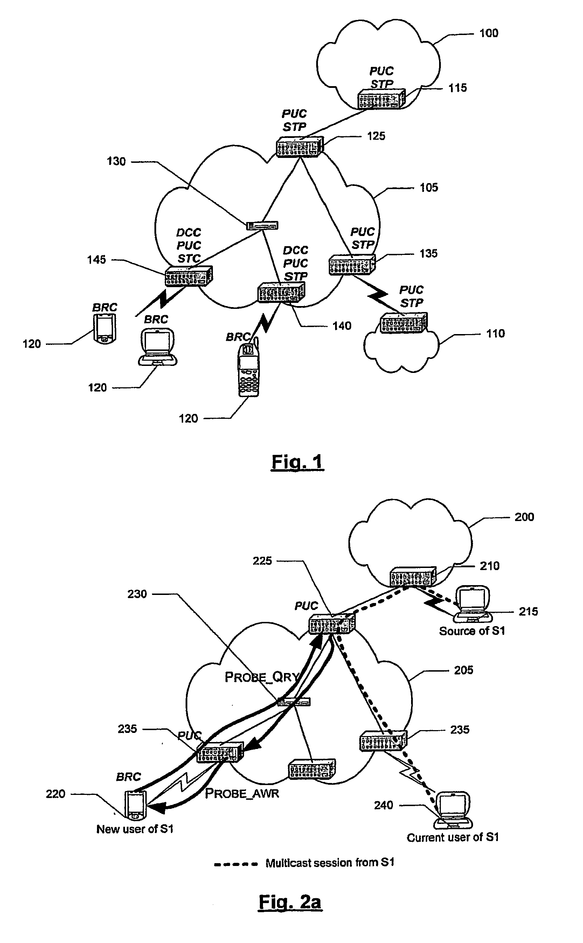

Fig. 1 schematically illustrates a network arrangement which can be used for multicast transmission. In Fig. 1 there are shown three different distinct networks 100, 105 and 110. They may be for example networks which are separated by different domains in the IP address space, or they may be three different types of networks, e.g. network 100 may be a LAN, network 105 could be a mobile network, and network 110 could be a wireless LAN network.

In network 100 there is located the source 115 of the distribution session which could be any server which is capable to distribute information "downstream", in other words towards the receiver(s). E. g. the source could be a video streaming server having different channels, a news server (also with different channels), or any information or service provider.

The receivers 120 are devices such as PCs, PDAs, mobile phones of the like which receive the downstream distributed information. On its way downstream in a multicast transmission a packet is distributed as long as possible only once, e.g. in Fig. 1 from source 115 to edge device 125 of network 105, and only where necessary. e.g. in device 125 the packet is duplicated and distributed separately to intermediate router 130 and to the other edge device 135. A further split into two downlinks is made at router 130 from where the duplicated packet then is separately transmitted to edge devices 140 and 145, in other words a packet is transmitted once over each link of the tree.

For clarification purposes it should be mentioned that in the following by multicast routing there is meant any method which sends only a single copy of the packet over each link of the tree, and not exclusively IP multicast standards.

As will be apparent from the foregoing, multicasting large amounts of data to a large number of receivers involves significant costs due to the fact that the multicast traffic is replicated in each branch point of the network. Since the network resources always are limited, e.g. in terms of bandwidth, and accordingly an increased usage of the network comes along with increasing costs and also possibly with less reliability, the network traffic caused by multicast routing is of significant concern and needs to be dealt with somehow. It should therefore be understood that if in the following the term "costs", "costs of a session", or "network costs" is used this term is not merely reflecting an economic aspect or concept but rather reflects the fact that the resources of a (each) network are limited, and that the accumulated resources reflect a certain value a part of which is used by each transmission thereby corresponding to "costs" in the sense of a fraction of the overall network "value".

It should be made clear that the foregoing concept of "costs" as a parameter corresponding to a fraction of the network resources must be distinguished from the more economic concept of "charging", in other words billing a user or assigning a certain "cost" to a corresponding monetary value.

Hence, it is important to make a distinction between the concepts of charging and cost: charging refers to the amount of payment extracted from a customer. Chargingschemes are based on costs, and other issues such as market structure and prices stability. On the other hand, costs refers to the value given by an operator to network resources. The present invention is directed to a method for monitoring, controlling and managing the costs involved with multicast sessions of network traffic. Based thereupon the provider or operator of a network may then apply his specific charging scheme.

Just as an example there may be in the future a range of network services, from basic to enhanced ones: basic services may be similar to current best-effort services, with universal connectivity and no assurance of quality. Enhanced services provide assured or guaranteed quality levels.

In this context, it is up to each provider to define the charging scheme to be used in its network. E.g. there may be provided to the users a distinction between basic and enhanced network services. This could mean that there is a charging scheme in which users pay a flat-rate for basic services and have access to enhanced services such as higher bandwidths on demand. By flat-rate it is meant that users pay only to subscribe a service, independently of the traffic that they create or consume.

Usage-based charges are applied when clients pay only for created and consumed traffic. Usage-based charges can be determined statically or dynamically: the former method depends on factors such as the time of the day and destination, while the latter depends on the current demand of resources and the overall network load.

There are several approaches that describe cost allocation schemes suitable for multicast traffic. In Herzog et al., "Sharing the "cost" of multicast trees: an axiomatic analysis", IEEE/ACM Transactions on Networking, 5 (6): 847-860, December 1997, there is proposed a scheme called Equal Link Split Downstream, which splits costs among receivers. However, this scheme has several drawbacks, including that it requires changes in all routers of a network, and does not explain how to cope with the dynamics of multicast sessions.

Henderson et al., in "Protocol-independent multicast pricing", in Proc. of International Workshop on Networking and Operating System Support for Digital Audio and Video (NOSSDAV), Chapel Hill, North Carolina, USA, January 2000, describe a scheme that allows a flexible distribution of costs between receivers of sessions in networks with a single level of service. There is proposed a mechanism based on servers placed at each domain to compute and distribute the cost of each session. However, it does not explain the mechanism used to split costs among receivers. Additionally, this approach is not suitable to be used in networks that can provide services with different quality levels, and the server placed in each domain represents a single point of failure.

In Carle et al. "Charging and Accounting for QoS-enhanced IP multicast", in Proc. Of IFIP 6th international Workshop on Protocols for High-Speed Networks", Salem, Massachusetts, USA, August 1999, there is proposed a layered framework for charging multicast traffic in ATM networks. Charging information is sent in ReSerVation Protocol RSVP messages, which are merged in the path from receivers to the sender. Although costs can be assigned to senders, based on the information included in reservation messages, this framework does not describe how to split costs among receivers. Moreover, this approach also requires changes in all routers.

Regarding the adaptation of the reception quality, in McCanne et al. "Receiver driven Multicast", Proc. Of Symposium on Communications Architectures and Protocols (SIGCOMM) , Palo Alto, USA, August 1996, there is disclosed a Receiver-driven Layered Multicast (RLM) mechanism, which was the first receiver-driven adaptive mechanism. Based on parameters such as packet loss the number of channels included in a session is increased as long as the quality of service indicated by the number of packet losses does not show any limit. However, RLM does not consider any budget considerations of a user when determining an upper quality threshold, and moreover has a slow convergence time due to its gradual approach of increasing the number of channels.

To define its charging scheme, operators need information about transmission costs to calculate usage-based charges. Moreover, accurate information is useful to periodically update their flat-rate schemes. Accordingly it is an object of the invention to allow operators to gather the required cost information which they may then apply to their individual charging schemes to calculate the charges for the user.

EP-A-1 341 341 discloses an apparatus for calculating a cost of receiving multicast data from a multicast session. A multicast network includes at least one multicast service, each multicast service including at least one multicast session. The apparatus receives a request to establish a connection to the multicast session, stores a start time for the connection and an end time for the connection and, after termination of the connection, calculates the cost of receiving the multicast data. The apparatus can receive a subsequent request to extend the connection, the subsequent request specifying a new end time for the connection, and store the new end time for the connection. Alternatively, the apparatus can receive a subsequent request to terminate the connection, the subsequent request specifying a new end time that precedes the end time for the connection, and store the new end time for the connection.

### SUMMARY OF THE INVENTION

The present invention is defined in the independent claims. The dependent claims define embodiments of the invention.

The rate adaptation system by multimedia applications according to an embodiment of the present invention tolerates a degree of quality degradation. However, the degree of tolerance depends on the costs of the multimedia session. Hence, it is important to control the rate adaptation while considering how much the user is willing to pay, in other words to consider his budget. Moreover there is the need for obtaining information about transmission costs in the network

According to one aspect of the invention there is provided a method of probing a network to obtain information about the unitary cost of a hew session in a multicast network, said method comprising: transmitting a probe query message containing information about said new session from a downstream device towards an upstream device located in the direction of the source of the session to obtain a unitary cost for said session; receiving said probe query message by said upstream device, if said upstream device is already receiving traffic for said session, updating the local information of the session with the address of the downstream device, and estimating the unitary cost of the new session; returning a probe answer message back to the source of the probe query message to return information about the initial unitary cost of the session for the source of the probe query message.

The unitary cost obtained by this embodiment can be used to calculate the costs of a session given the channels and the transmission rate of the individual channels.

According to one embodiment the method may comprise: If said upstream device is not yet receiving traffic for said session, storing information about the channels of the new session in addition to the information about the address of the downstream device, and forwarding the probe query message further into the direction of the source of the session.

This enables too find an upstream device where the session is already running, thereby making a multicast transmission for the new session possible.

According to one embodiment the method may comprise: estimating said unitary cost based on the total cost for the total available bandwidth in said upstream device and a unitary rate as a fraction of said total available bandwidth, said unitry cost being the transmission cost per unit of bandwidth.

This enables the determination of the unitary costs under the given economic boundary conditions at a cerain device in the network.

According to one embodiment the method may comprise:
returning said probe answer message into downstream direction, whereas in each downstream device through which said probe answer message passes on its way towards the destination of said new session said unitary cost is updated.

The "travelling" of the probe anser message and its updating at each network device is an efficient and accurate way of estimating the total unitary cost from a network device where the session already exists down to the destination.

According to one embodiment the method may comprise: adding the local unitary upstream cost as received through the probe answer message and the local unitary downstream cost to obtain a total unitary downstream cost.

According to one embodiment the method may comprise: said check whether said upstream device is already receiving traffic for said session, said estimation of said unitary cost and said generation of said probe answer message is only performed network devices located at the edgde of a network.

This makes it possible that the mechanism for estimating the unitary cost needs only to be implemented in the edge devices of the network such that a large part of an existing network infrastructure like intermediate routers can be used without any change.

According to one embodiment the method may comprise: intermediate network devices which are not located at the edge of a network are transparently forwarding said probe query messages and said probe answer messages.

Transparent transmission ensures that the intermediate network devices are merely forwarding the messages related to the cost estimation process but do not actually "process" them in the sense that they have to recognize and process their content.

According to one embodiment the method may comprise:
said probe query message, said probe answer message, and said unitary cost information are transported by one or more empty fields of an existing network protocol different from the fields reserved for the payload transmission.

This is an efficient way of using existing network protocols to forward the messages and information relevant for the cost estimation process through devices like intermediate routers in an existing network infrastructure.

According to one embodiment the method may comprise: receiving a probe answer message containing information about the unitary costs of a session from an upstream device, updating the unitary costs of the session transported by the probe answer message in the device receiving said probe answer message; and forwarding said probe answer message towards the destination of said session.

According to one embodiment the method may comprise:
in each upstream device receiving said probe query message, updating session information to store information about said new session, its destination and its channels as well as information about the downstream device from which said probe query message was received.

The updating of the information about ongoing or newly to be established sessions is helpful to make a cost distribution about multiple users possible. Moreover, it keeps the network devices informed about ongoing sessions and sessions which are to be established.

According to one embodiment the method may comprise: if an upstream device receives a probe query message while not receiving traffic for said new session identified by said probe query message, setting a timer, and if said timer runs out for said new session, erasing the information stored for that session if the rate for that session is still zero.

This ensures that the network devices are not flooded with information about sessions which are either not active anymore or which even never have become existent (e.g. due to budget problems).

According to one embodiment the method may comprise: the information identifying a session and the information about the costs of a session are only stored in edge devices of said network.

This keeps the need for amendments in existing networks low when implementing an embodiment of the invention.

According to one embodiment the method may comprise: among the devices within a network only the edge devices generate the probe query message and the probe answer message. This also keeps the need for amendments in existing networks low when implementing an embodiment of the invention.

According to one embodiment the method may comprise: a method of receiver-based controlling the reception data rate of a session which may include multiple channels and wich is been distributed to said receiver over a multicast network, said method comprising: based on a unitary cost and the rate of each channel as well as based on a budget for transmission costs, determining the number of channels as allowed by the budget, andruning only as many channels for the session as are allowed based on the budget considerations.

This makes an instantaneous adjustment of the channel selections based on cost considerations possible. The unitary cost may be determined by the methods according to previous embodiments.

According to one embodiment the method may comprise: allowable channels are determined based on said budget and further based on the priority of the individual channels. This takes into account priority choices made by the user.

According to one embodiment the method may comprise in response to an initiation signal to start a session at the destination location, generating a probe query message and forwarding the probe query message towards the source of the session to query about the unitary cost of the session; receiving an probe answer message from an upstream device located into the direction of the the source of the session and containing information about the unitary cost; based on the received information, subscribing as many channels as allowed by a budget determined for said session, starting from the channels with highest priority.

According to one embodiment the method may comprise:receiving repeatedly a cost adjustment message containing updated information about the costs of the session at the destination of said session; if said updated costs are lower than the budget for said session, subscribing additional channels as allowed by said budget; if said updated costs are higher than the budget, unsubscribing channels from said session starting with channels having the lowest priority until the budget criterion is met.

This allows a continous updating of a suitable selection of channels.

According to one embodiment the method may comprise:the network is able to handle different classes of service, a session belonging to a certain class and a class having assigned a total value and a corresponding total bandwidth, and wheres for a certain session the unitary cost is estimated dependent on the class to which the session belongs.

This takes into account that the network may be divided into different classes for which different values are assigned to a unit of bandwidth.

According to one embodiment the method may comprise: forwarding to a downstream device in each downstream link in said network a cost adjustment message containing information about the transmission costs in said respective downstream link, and said downstream device updates its upstream costs based on said received cost adjustment message.

According to one embodiment the method may comprise: said cost adjustment message is forwarded to said edge devices located into the direction of said downstream links, and said edge devices treat said cost adjustment message as providing information about the upstream costs for said downstream edge devices.

According to one embodiment the method may comprise: said cost adjustment message contains cost information for each session in said downstream link separately.

According to one embodiment said method may triggered by one or more of the following: a cost adjustment message from an upstream device;
a cost leave message from a downstream device informing about the cancellation of a channel or session; a run-out of a timer being repeatedly set to ensure a repeated update of the cost information in said network device.

According to one embodiment there is provided a method of distributing in a multicast transmission network the transmission costs among customers whose terminals are connected to an edge device of said network, said method comprising:distributing the costs of a distribution session among the customers that subscribed it based on the reception rate used by each customer.

According to one embodiment the method may comprise: dividing the estimated cost of the session in the access link among the channels of the session based on the ratio between the rate of each channel and the total rate of the session; dividing the cost of each channel equally among each customer using it. This enables accumulation of the distributed channel costs for each customer based on the channels subscribed by this customer.

According to one embodiment the method may comprise: forwarding the total cost obtained for each session to each customer device in a cost adjustment message; and using said total costs information as an input to reception data rate control method according to another embodiment

According to a second aspect of the invention there is provided an apparatus for probing a network to obtain information about the unitary cost of a new session in a multicast network, said apparatus comprising: a transmitter for transmitting a probe query message containing information about said new session from a downstream device towards an upstream device located in the direction of the source of the session to obtain unitary costs for said session; a receiver for receiving said probe query message by said upstream device, if said upstream device is already receiving traffic for said session, updating the local information of the session with the address of the downstream device, and estimating the initial unitary costs of the new session; a responder for returning a probe answer message back to the source of the probe query message to return information about the initial unitary costs of the session for the source of the probe query message.

According to further embodiments there are provided apparatuses comprising elements or units to carry out methods according to embodiments of the invention.

### BRIEF DESCRIPTION OF THE DRAWINGS

Fig. 1 illustrates a network configuration according to an embodiment of the invention.
Fig. 2a and Fig. 2b illustrate schematically the signalling in embodiments of the invention.
Fig. 3 shows a flowchart illustrating an embodiment of the invention.
Fig. 4 shows a flowchart illustrating a further embodiment of the invention.
Fig. 5 shows a flowchart illustrating a further embodiment of the invention.
Fig. 6 shows a flowchart illustrating a further embodiment of the invention.

### DETAILED DESCRIPTION

In the following embodiments of the present invention will be described by referring to the accompanying drawings.

The embodiments of the present invention relate to multicast transmission in networks. The embodiments relate to different elements which themselves are closely related. These elements or mechanisms are a budget based rate control (BRC), the probing of unitary costs (PUC), the splitting of transmission costs (STC) and the distribution of costs per customer (DCC). These mechanisms are described in more detail in the following, whereas the aforementioned abbreviations may be used to refer to these mechanisms.

According to one embodiment there is provided a mechanism for executing a budget-based rate control for a session. A session may e.g. be a video session comprising several channels of different thematic content. Users may by using a multimedia application running on their terminal subscribe at their terminals to such a session, e.g. by identifying the channels which they wish to get delivered and by identifying the source which should deliver the session comprising the channels. They may further identify or assign priorities to the individual channels selected for the session which they subscribe. Alternatively the priority of one or more of the channels may be predefined. If the user has already a session running or if he wishes to open more than one session it may according to one embodiment also be possible to assign priorities to the respective sessions. Once the session has been defined and subscribed by the user the further procedure according to an embodiment of the invention is as described in the following.

Once a session is subscribed by a user there is obtained the unitary cost for this session. This is for example done by a probing mechanism for probing the unitary cost which will be described later in more detail. From such a probing mechanism there is obtained a cost parameter, e.g. the "unitary costs" for the session. Based on the thus obtained "unitary cost" which may be e.g. the costs for the transmission of a bit per second for this session, the total costs of the session as defined by the user may be calculated. This calculation may be carried out based on the rate of the individual channels which belong to the session. Therefore based on the definition of the session as subscribed by the user, namely based on the selected channels and their rate there is calculated the total cost of this session based on the unitary cost which gives the cost for a certain unitary bitrate (e.g. 1 bit per second).

The calculation of the total costs can be carried out in the network device which requested initiation of the session, in other words the destination of the session or the final downstream device in the downstream path starting at the source device. Once the "destination device" or "receiving device" has received the unitary cost parameter from an upstream device in response to its probe query message it starts to calculate the total costs of the session.

If the total cost of the session as defined by the user would exceed the limit (the "budget") of the user a rate adaptation mechanism begins to operate the purpose of which is to "control the cost" or in other words ensures that the costs involved with the session will remain within a budget. For that purpose the adaptation mechanism subscribes only as many channels as can be distributed without exceeding the budget. Preferably this is done by starting with the channels which have the highest priority.

With such a mechanism the transmission rate for a certain session can be instantaneously (without any delay) adapted to the budget limitations of the user, thereby avoiding the slow convergence time involved with a mechanism like the receiver layered multicast (RLM) mechanism which is driven by packet losses as parameter. Moreover, the RLM mechanism doesn't take into account budget considerations at all.

In one embodiment the number of channels is from time to time adjusted, e.g. based on updated information about the sessions cost. This updated information may be provided by such mechanisms as the "distribution of cost per customer" to be described later.

Fig. 1 schematically illustrates a network configuration according to an embodiment of the invention. In the Figure there is shown which elements are implementing and operating in which parts of the network. As can be seen from Fig. 1, the BRC mechanism is implemented in the receiver. The BRC mechanism receives as an input the unitary cost which is delivered from an upstream device, and therefore as long as the network is able to provide the unitary cost for a session upon a request the BRC mechanism needs only to be implemented in the receiver without any further modifications in the rest of the network.

.Fig. 1 also shows for other mechanisms such as PUC or STC and the DDC in which network elements they need to be implemented.

According to one embodiment a receiving device may subscribe and maintain more than one session simultaneously. In such a case there may either be a budget for each session separately or there may be established an overall budget for the total costs of all sessions. In the first case the system operates as described before by determining for each session individually the allowable channels such that the budget is not exceeded. In case of an overall budget the channels also may be determined based on the priority of each channel. Alternatively other selection schemes for the channels may be employed, e.g. such that based on a ranking of the sessions for each session on after another (starting with the session having the highest priority) the channel with the highest priority for this session is selected, then for the next session also the channels with the highest priority is selected, and so on until for the session with the lowest priority there is selected the channel having the highest priority. If the budget is not yet exceeded, then the procedure continues for the highest priority session selecting the second most important channel, and so on, until allowing a further channel would exceed the budget.

It should further be mentioned that the network may provide services of different classes (e.g. different quality levels), and in this case a session may belong to a certain class and it may be determined for each class separately a respective unitary cost. Typically higher quality services need more network resources and therefore for a service belonging to a "higher class" the unitary cost may be higher.

In the following the mechanism for probing the unitary cost (hereinafter also referred to as PUC) according to one embodiment will be briefly described.

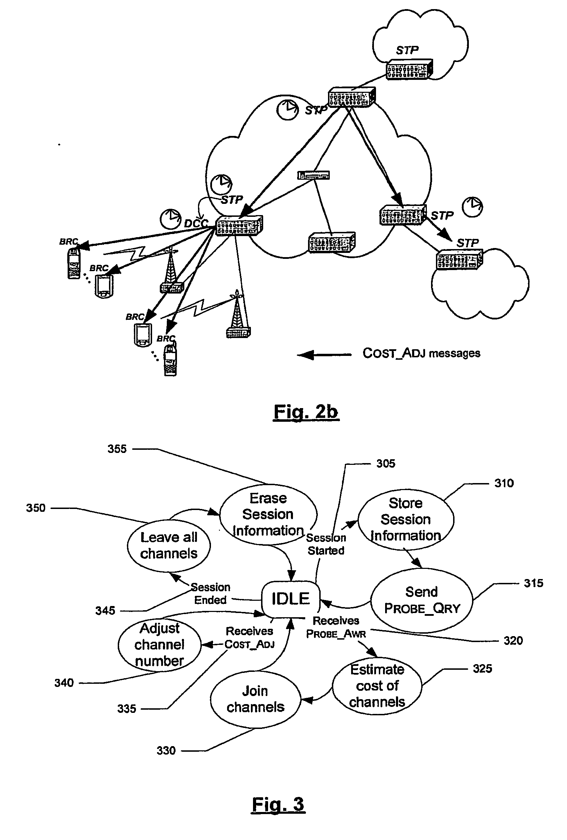

The PUC mechanism provides control functions in terminals (receiving devices which are "targets" of a new downstream session) with information about the approximated unitary cost of a new distribution session. For this, the PUC mechanism probes network devices at the edge of networks between the customer and the first network device where the session is present. Referring to Fig. 2, there is briefly illustrated the PUC mechanism. There is an ongoing session S1 between source 215 and receiving device 240. This session is distributed via the (edge) device 225 and the other (edge) device 235 to the receiver 240. A user at device 220 now wants to open a new session and defines its desired source (device 215) and the channels, possibly (if not predefined) also the priority of the channels. By chance this new session may no be identical (or be a subset of) the already existing session S1 which in principle would make the use of multicasting possible. Then there is generate the query message PROBE_QRY which is forwarded towards the source device. At the first edge device 235 where the query arrives there is no ongoing session which is identical (at least partly) to the requested one. Therefore the query message is forwarded further in the upstream direction to search for a node in the network where an (at least partly) identical session is already running. After having passed also through intermediate node 230 the query message arrives at (edge) node 225 where the same session is already running. This means that node 225 can be used as a branch point for a multicast transmission and the (local) costs for the session can be determined at this node. The unitary cost for the session resulting from this determination is returned through a probe answer message (PROBE_AWR) the same path back to inform the receiving device about the unitary cost of the session.

The check whether a same session is already running in a network device which receives a Probe_Qry message can e.g. be done based on the session identifier which identifies the session. In this context a session as offered by a source may comprise a certain set of channels which are predefined, and this set of channels is then assigned a "session identifier". This session identifier is then used by the receiving device where the new session is to be initiated and it is assigned to the session to be newly generated. It should be noted here that this does not necessary mean that the session which is to be newly established must comprise all of the channels which belong to the "whole session" as offered by the source, rather it may also be possible that a user chooses only some of these channels as offered by the source under this session identifier to form and define the session which is to be newly established. However, the newly to be established session needs to comprise least a "subset" of the "whole session" as offered by the source under the certain session identifier. Just as an example, a session having the session identifier S1 and offered by a source X may comprise channels 1, 2, 3, 4, 7, 8, and 9, and this information is communicated to the receiving device that such a session is available for delivery. A user may then choose this session to be newly opened in his receiving device, and he may then choose e.g. channels 1,2, and four as the channels which he wishes to be contained in his session, and this session still is identified by the session identifier S1. The session identifier may then be used by a network device when checking in response to the reception of a Probe_Qury message whether in this network device such a session is already running (and delivered to a different destination). If so, then the Probe_AWR message containing the local unitary cost is generated and returned. According to one embodiment it may also be possible to use a channel identifier to identify a session, e.g. by using the first channel of the session or a channel which by convention is used as channel identifier.

Based on the received answer message and the cost information the receiving device then is able to determine the (actual) cost of the session and to use only as many channels as are allowable without infringing the budget of the user.

According to the foregoing example at the intermediate node 230 no session identical to the requested one was running and therefore the query message was forwarded further into the upstream direction. However, according to one embodiment even if in intermediate device 230 an identical session would have been running nevertheless the query message would have been further forwarded upstream without generating a response in intermediate device 230. According to this embodiment within a network only edge devices such as devices 225 and 235 generate query messages (and answer messages). This makes it possible that for intermediate nodes like node 235 no modification in an existing network is necessary when implementing the present embodiment. Intermediate nodes must only have the capability to (transparently) forward the query and answer messages, and this can be done e.g. by using empty fields in existing network protocols which are not evaluated by intermediate nodes.

It should be mentioned that the query messages are also used to inform network devices about the characteristics of a new distribution session such as the destination, the source, and the channels belonging to the session.

The PUC mechanism probes the network to get information about the unitary cost of distribution sessions, allowing budget-based adaptation mechanisms (e.g. the BRC mechanism) to decrease the convergence time required to reach a good reception quality level, while respecting the budget of the customer.

It should be noted here that the unitary cost sent to a customer (user or network) may reflect the charging scheme agreed between the customer and its network provider. This means that the cost information exchanged between a provider and a customer may be modified by the provider's charging mechanism. Also, if the probing message (and its corresponding answer) travel beyond the border of a certain network or domain this may also influence the unitary cost transmitted in the answer message to reflect e.g. charging schemes which are in place between different providers for sessions traversing their networks.

According to one embodiment the probe query message is forwarded upstream up to a device where a corresponding session is already running. If there is no ongoing session between the source and any terminal of a user the probe query travels in upstream direction up to the edge device of the network located most closely to the source. Between this edge device and the source the session is already running because in a standard multicast environment a source of a multicast transmission offering a session establishes said session at least with the edge device of a network from which the data then is further distributed into the downstream direction. If no user or receiving device has ever requested the offered session yet the edge device of the network just drops the packets of the session it receives from the source. In case of the first probe query message received for a certain session therefore this network edge device generates the probe answer message and sends it into the downstream direction together with the (total) local unitary cost in this network edge device.

According to a further embodiment there is provided a mechanism for splitting transmission costs (hereinafter also referred to as STC) of multicast transmissions to downstream links. According to one embodiment this takes into account the dynamics of multicast trees in class-based networks. In such a network a network operator provides the capability to differentiate traffic in different network classes, each one offering e.g. a different quality level and having different costs.
The STC mechanism according to one embodiment splits the upstream transmission costs of multicast trees among downstream links, only at edge devices of class-based networks. It should be noted here that the term upstream transmission cost refers to the costs that a session has due to its transmission from the source until the current edge device. For this, the STC mechanism collects, in each edge device, information about the costs of a session provided by the upstream neighbour device, and splits that cost among downstream edge devices taking into account the total rate of the session (sum of the rate of each one of its channels), and the bandwidth and cost of the network class used by that session. In other words, the bandwidth and costs of the network class used by that session corresponds to the "total value" which the class (to which the session belongs) is assigned by the network operator, and the total rate of the session (which may be derived from a metering device) corresponds to a fraction of this total value by prorating the total rate of the session with the bandwidth of the class to which this session belongs.

It should be noted here that when neighbour devices are places at the edge of different networks, the costs communicated by the upstream neighbour to the downstream one may reflect the charging scheme agreed between the two network providers. This means that the cost information exchanged between a provider and a customer may be modified by the provider's charging mechanism. It should further be noted that the definition of the values for the bandwidth and cost of each network class is a policy issue and not relevant for the actual mechanism of distributing the costs (STC mechanism).
Instead of merely forwarding upstream costs to downstream devices the STC mechanism splits the cost of distribution sessions between downstream links of edge devices, thereby decreasing the session costs at each outgoing interface. This allows network providers to attract and keep customers by supplying them with a good trade-off between quality and costs.

According to a further embodiment there is provided a mechanism for the distribution of costs per customer (hereinafter also referred to as DCC). This mechanism distributes the costs of a distribution session among the customers that subscribed to it, based on the reception rate being used by each customer. For this purpose the DCC mechanism collects from terminals information about each session and distributes the costs of those sessions (provided by mechanisms such as the STC mechanism) among all customers receiving them, by taking into account the number of channels subscribed to by each customer and the rate of each channel.
It should be noted that the costs sent to a customer may reflect the charging scheme agreed between the customer and its access network provider. This means that the cost information exchanged between a provider and a customer may be modified by the provider's charging mechanism.

Referring now to Fig. 1 there is shown an exemplary network configuration where it is further illustrated which of the aforementioned components are installed at which part of the network. As can be seen from Fig. 1, only the BRC mechanism is to be installed in terminals, whereas the other mechanisms such as PUC, STC and DCC are installed in the network. Moreover, all the functionalities required by the PUC, STC and DCC mechanisms are located in edge devices, without any interference with interior routers. While the PUC and STC mechanisms are active in all edge devices, the DCC mechanism is only active at network edges that serve as access gateway to individual customers. This shows that the embodiments of the invention can be implemented without the need to modify all routers in an existing network, instead modifications are merely necessary in the terminals (receivers) and the edge devices.

Referring now to Fig. 2, there is illustrated the signalling used by the aforementioned mechanisms. Figure 2a) shows the signalling triggered by the BRC mechanism and used by PUC to collect information about the unitary cost of a multicast session, when new customers join it. Figure 2b) shows the signalling as used by STC and DCC mechanisms to propagate cost information. The STC mechanism propagates information about the cost of sessions in each downstream link, while DCC propagates information about the cost associated with each customer. As shown in Fig. 2b), the STC and DCC mechanisms according to one embodiment are triggered by timers, and the DCC mechanism uses as input the output of another mechanism, such as the STC, which splits the cost of each session among each downstream link where the session is present. A more detailed description of these mechanisms according to embodiments of the invention is provided in the following.

At first the BRC mechanism according to one embodiment will be described in somewhat more detail referring to Figures 2a and 3. It should be noted that if there is referred to hereinbefore or in the following to BRC, PUC , STC and DDC as "mechanisms", then it will be readily apparent to the skilled person that such "mechanisms" can be implemented a network device by suitably programming a microprocessor contained in such a device. Accordingly, such a mechanism can be either implemented by some software code causing a microprocessor and possibly its peripheral devices to act as described herein, or by a specific piece of hardware such as a gate array or any electronic device or integrated circuit which has been designed to act as described herein. In view of the foregoing comments the term "mechanism" as used herein is also to be understood as a method comprising a series of steps which when being executed correspond to the operation of a network device as described herein.

The BRC mechanism has three triggers, which are the reception of an application signal notifying about the start or end of a session, and the reception of Probe Answer (also referred to as Probe_Awr) messages and Cost Adjustment (also referred to as Cost_Adj) messages from the network. In response to such events actions as described in the following are performed:
In response to the reception of application signal to start a session:
   The subscription of a session (e.g. initiated by a user using a multimedia application running on his terminal) must include information about all channels which together define the distribution session (e.g. source and channels identifiers, priority of each channel, rate), and the customer's budget for the session. This corresponds e.g. to operation 305 in Fig.3. The source and channel identifiers may be IP addresses, as it happens when IP multicast is used to create the distribution trees.
   These informations are received and stored by the BRC (operation 310 in Fig. 3) which according to one embodiment can be implemented as a software component installed and running on the terminal of the user.

After storing the information about the session, the BRC mechanism sends Probe Query (hereinafter also referred to as Probe_Qry, operation 315 in Fig. 3) message to the source of the multicast session to query about the approximated unitary costs of the session. This is e.g. also illustrated in Fig. 2a) which shows the Probe Query message originating from device 220 the user of which newly subscribes to an already existing session S1 used by the user of device 240. The purpose of the Probe Query message going upward is to trigger the return of a message containing information about a unitary cost, a so called Probe Answer (hereinafter also referred to as Probe_Awr) message.

### Reception of a Probe_Awr message (operation 320 in Fig. 3):

This message brings information about the approximated unitary cost of a session (the way how this information is actually gathered in the network is explained later in connection with the PUC mechanism. Based on this unitary costs for each channel and the rate of each channel (which has been stored already in response to the start session signal), the BRC mechanism estimates the costs of each channel (operation 325 in Fig. 3). After this, BRC subscribes as many channels as allowed by the customer's budget (operation 330 in Fig.3), starting from the channels with highest priority.

### Reception of a Cost Adjustment (Cost_Adj) message:

Periodically, the BRC mechanism receives a Cost_Adj message (operation 335 in Fig. 3) with updated information about the cost of a session (the way cost information is updated in the network is explained later in more detail in connection with the STC mechanism. The received updated cost information is used to adjust the number of channels (operation 340 in Fig. 3). This according to one embodiment happens in the following way:
a) If the updated cost is lower than the customer's budget for that session, the BRC mechanism subscribes new channels as long as allowed by the budget value (channels are subscribed starting from the most important one). The number of channels to be subscribed is computed based on the unitary costs of the session and the rate of each channel.
b) If the updated cost is higher than the customer's budget, the BRC mechanism unsubscribes channels as long as the costs are higher than the budget value (channels are removed starting from the less important one). If the budget is not enough to keep the basic channel of the session, a signal is sent to the application asking the user to increase the budget. If budget is not increased, the BRC executes the actions to leave the session, as explained in the following. Otherwise, steps a) and b) as explained above are repeated.

### Reception of application signal to end session:

The BRC may (operation 345 in Fig.3) generate un-subscribe messages (may be according to one embodiment IGMPv3 or MLDv2, depending if the system is IPv4 or IPv6, respectively) to leave all channels of the session (operation 350) and to erase all information (operation 355) about that session. In the application signal, the session may be identified by the session identifier which may either be a dedicated identifier or-depending on the embodiment-be also a channel identifier which e.g. y convention identifies a certain session.

When the BRC mechanism subscribes a channel, the identifier of the channel and optionally the rate of the channel (if not predefined or invariable) are included. The information about the channels (e.g. by using channel identifiers) together with some information identifying the session has to be delivered upstream in order to trigger the return of a response which delivers information about the unitary cost of the session. In other words there must be provided a mechanism which allows the transport of this information, preferably by already existing networking mechanisms or protocols.

As the present invention relates to multicasting, in the network where the present invention is to be implemented there is already existing some mechanism to build multicast trees. An example would be using IGMPv3 or MLDv2 in the access points for Ipv4 and Ipv6, respectively. IGMPv3 is described in Cain et al., "internet Group Management Protocol, version 3, RFC3376, IETF, October 2002", and MLDv2 is described in "Vida and Costa, Multicast Listener Discovery Version 2 (MLDv2) for Ipv6, RFC3810, IETF, June 2004".

For the purpose of transporting the data as mentioned before, the auxiliary data field of IGMPv3 and MLDv2 reports may be used, as well as SIP (Session invitation protocol) INVITE messages. IGMPv3 and MLDv2 reports which are standards for group messages allow routers to collect information about individual users, since the 'host suppression' mechanism, present in previous versions, was removed. In any case, the message used to subscribe a channel should include also the identifier of the most important channel of that session. To un-subscribe a channel, the BRC mechanism may use IGMPv3 and MLDv2 reports or SIP (Session Invitation Protocol) BYE messages (including the identifier of the channel to be unsubscribed). Alternatively other protocols or data fields of other protocols may be used.

The BRC mechanism in one embodiment can be combined with rate-based adaptation mechanisms, such as the RLM, which will adjust the number of channels based on the percentage of packet loss. In this scenario, one possible combination would be to have BRC controlling the increase of reception rate, and a RLM-alike mechanism controlling the decrease of such rate (if the packet loss is getting too high).

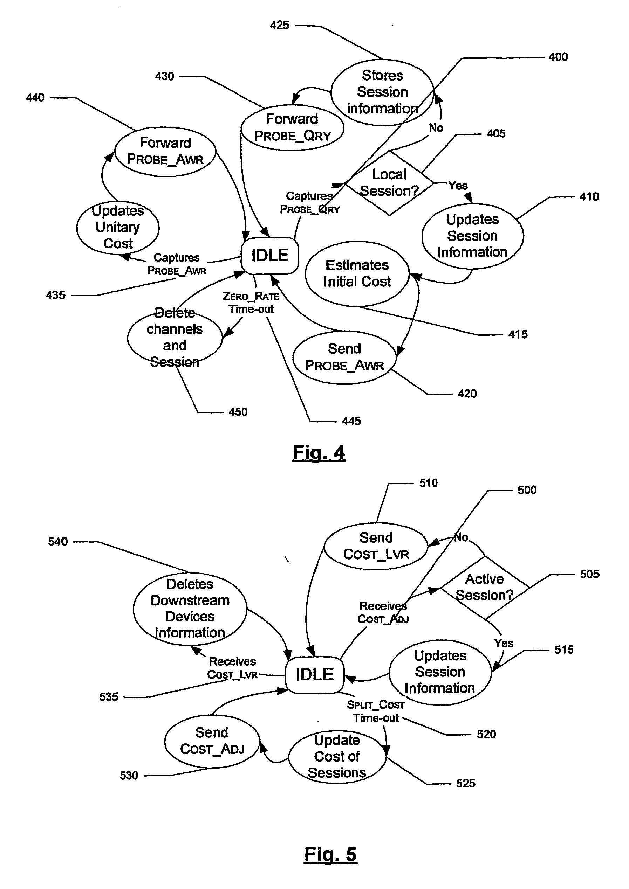

The BRC mechanism needs for its operation information about the unitary cost of a session. This information is provided by the PUC (Probing Unitary Costs) mechanism which will now be described in more detail in the following referring to Fig. 4.

The PUC mechanism according to one embodiment has three triggers, which are the reception of Probe_Qry and Probe_Awr messages, and the run-out of the a timer referred to hereinafter as Zero_Rate timer. After one of those events the actions as described in the following are performed.

### Capture of a Probe_Qry message from a downstream device:

In response to the reception (operation 400 in Fig. 4) of a Probe_Qry for a session (identified by e.g. its session identifier ) the PUC mechanism checks (operation 405) if this device on which the PUC is running and which captured the Probe_Qry is already receiving traffic for such a session (in other words, is there such a local session running on this device). If so, PUC first updates (operation 410) the local information of the session with the address of the downstream device from which the Probe_Qry was received. Thereafter it estimates the local unitary cost of the session in the link where the Probe_Qry was received based on the local unitary rate of the session (e.g. 1 bit per second), the bandwidth allocated to the class used by the session and the cost of that class. Just as an example, assuming that the (total) bandwidth of the class to which the session belongs would be 1 Gbit/s, further assuming that the corresponding (total) cost would be 2000 EUR, then the unitary cost of 1 bit/s for a session in this class would be 2000/10⁹ EUR.

In order to obtain the "total" unitary cost of the session the previously obtained "local" unitary cost of the session in the link through which Probe_Qury was obtained is added to the "unitary upstream cost" of that session. This unitary upstream cost is the cost which is generated by the data flowing from an upstream direction into the device where the Probe_Qry message has been received. The unitary upstream cost is calculated based on the upstream cost and the rate of the session (which may be determined from a meter which is located at the upstream interface and measures the incoming data rate). By dividing the upstream costs (which are already stored in the device due to the already existing session) by the data rate the unitary upstream cost may be obtained. These unitary upstream cost added to the local unitary cost (in other words the "unitary downstream cost) give the total local unitary cost for the session in the device which has received the Probe-Qry message.

The resulting total unitary cost is then included into a Probe_Awr message which is sent back to the source of the Probe_Qry message. According to one embodiment all messages are sent with the IP alert option set, in order to be captured by the PUC mechanisms installed in the path until their destination.

If operation 405 results in that the device is not yet receiving traffic for that session, the PUC mechanism stores (operation 425) information about the channels of the session in addition to the information about the address of the downstream device.

According to one embodiment furthermore information about the incoming interface may be stored. The PUC mechanism then forwards the Probe_Qry message into the direction of the source of the distribution session. After any of these actions, the PUC mechanism sets the Zero_Rate timer. If the Probe_Qry message on its way upstream is received by an upstream edge device the procedure in this device is carried out in the same manner as described hereinbefore.

### Capture of a Probe_Awr message from an upstream device:

In response to the reception (operation 435) of a Probe_Awr for a session (identified by its most important channel) the PUC mechanism updates the unitary cost of the session transported in the Probe_Awr message, and forwards the message towards its destination. For this purpose it estimates the local unitary cost for the downstream link starting from the device where the Probe_Awr message was received. This is - like described in the previous case in connection with the reception of the Probe_Qry message - done based on the bandwidth allocated to the network class to which the session belongs, based on the corresponding cost or value assigned to this bandwidth, and based on the unitary (downstream) rate of the session. This gives the local unitary cost of the session, and to obtain the total unitary cost it has to be added to the unitary upstream cost. This unitary upstream cost, however, was received as information contained in the Probe-Awr message and is identical to the value of the total unitary cost which was obtain at the device located one stage more in the upstream direction.

In other words, when the Probe-Qry message is received on its way in upstream direction for the first time at a device where the same session is running then at this device the total unitary cost in downstream direction at this device for the newly to be established session is estimated by adding the local unitary upstream cost plus the local unitary cost in downstream direction. The local unitary upstream cost can be obtained by dividing the rate of the already ongoing session coming in from the upstream direction (which can be measured by a meter) by the upstream cost of the already ongoing session. The latter information is available in the device because it keeps track of the costs of an ongoing session. Alternatively the local unitary upstream cost may be retrieved as it has once been delivered to the device by a Probe_Awr message at the time when the now already ongoing session was initiated. The local unitary (downstream) cost is estimated based on the total bandwidth and the total value assigned to it and based on a unitary rate (e.g. 1 bit/s). The total unitary downstream cost then is the sum of the local unitary upstream cost and the local unitary downstream cost in that device.

Once the total unitary (downstream) cost has been calculated at this device (by adding the local unitary cost to the local unitary upstream cost) it will be forwarded in downstream direction, and at each (edge) device in downstream direction the total unitary cost will be updated by adding the local unitary downstream cost to the value of the local unitary upstream cost which was just received through the Probe_AWR message from the upstream direction. This procedure continues into the downstream direction until the Probe_Awr message (and the total unitary cost transported and continuously updated by it on its way in downstream direction) are received by the terminal which has requested the initiation of the session and which can then based on the total unitary cost select the suitable channels for the session to keep the budget.

### Run-out of the Zero_Rate timer:

when this timer runs-out for a specific session (operation 445), the PUC mechanism erases the information stored for that session (operation 450) if the rate of the session is still zero. This cleans up from time to time the records in the devices about sessions which actually are no more active, or sessions which may even never have become active (e.g. due to budget limitations).

In the following the splitting of transmission costs between downstream links will be described in somewhat more detail by referring to Fig. 5, which gives a detailed description of the operation of the STC mechanism. This mechanism in one embodiment has three triggers, which are the reception of Cost_Adj and Cost_Lve messages, and the run-out of the Split_Costs timer. After such events the following actions are performed.

### Reception of Cost_Adj message from an upstream device:

After the reception of a Cost_Adj message (operation 500 in Fig. 5) for a set of sessions (identified by their session identifiers) the STC mechanism checks (operation 505) if those sessions are still active in the device. For all the already absent sessions, the STC mechanism sends a Cost Leave Cost_Lve message (operation 510) to the source of the Cost_Adj message. For all sessions that are still locally active, the STC mechanism updates (operation 515) the local information about the upstream cost of those sessions.

### Run-out of the Split_Costs timer (operation 520 in Fig. 5):

when this timer runs-out, the STC mechanism performs an update (operation 525) the costs of the sessions in the device by performing the following actions:
a) for each session: split their upstream cost among the downstream links where the session is present. An example of a possible computational process is the one that considers the ratio between the upstream rate of the session and the rate of the session in each downstream link. Assuming the rate of the upstream link (i.e. the rate with which the session enters the device from the upstream direction) is 100, assuming that downstream link A has a rate of 80, downstream link B a rate of 70 and downstream link C a rate of 100, then the upstream costs are split among the downstream links A, B, and C such that A gets a fraction of 80/100, B gets 70/100, and C gets a fraction of 100/ 100 of the upstream costs. The rate of a session in a downstream link is estimated based on the rate of each of its channels, a value that is collected from the meter present in that link. The upstream rate of the session can be collected in the Cost_Adj message or from a meter installed in the upstream interface of the edge device.
   As an alternative it would also be possible to distribute the costs such that in the previous example A gets a fraction of 80/250, B gets 70/250, and C gets 100/250. This takes into account the advantage which the network provider has through the use of multicasting, and it forwards this advantage directly to the customer. In other words, the more people are participating in a multicast session the less network resources are needed (compared to serving the same number of people by unicast transmission), and consequently the costs can be distributed among more users.
   Another embodiment could distribute the costs based on and the ratio between the upstream cost of the session and the number of downstream links where the session is present, this would in the previous example lead to 1/3 of the upstream costs for downstream links A, B, and C.
b) for each downstream link:
   estimates the local cost of each distribution session in that link based on the local rate of the session, the bandwidth allocated to the network class used by the session and the cost of that class. It should be noted here that the mechanism used to set the bandwidth and cost of a class for each link of each edge device is a policy matter and does not influence the operation as described in principle herein. Just as an example of numbers, assuming that the bandwidth allocated to the network class used by the session is 200 and the value of the class is 100, further assuming that the session uses a rate of 4, then the local cost of the distribution session for that downstream link would be 4/200 * 100 = 2.
   After this determination, the total cost of a session in a downstream link is computed by adding the local cost of the session in the link and the share of the upstream cost of that session (the value that was computed in step a)).
c) for each edge device, in each downstream link:
   It should be noted that the STC mechanism collects information about each downstream edge device (its address and the information about the number of channels of each session present in such a downstream device) by means of a probing mechanism such as the PUC described hereinbefore. This information can be used to more accurately distribute the costs of the downstream links.

According to one embodiment the transmission costs of each session in each downstream link, as computed in step b), is divided among all downstream edge devices which run this session. For instance assume that the determination of the costs of the downstream link from device 125 to device 130 in Fig. 1 resulted in a value of 100, and the costs for the downstream link from device 125 to device 135 resulted in a value of 50. The resulting total downstream link costs would be 150, and because device 125 is aware of the fact that devices 135, 140 and 145 run the same sessions device 125 may distribute the total costs of 150 among these three device, e.g. by taking into account the number of channels used in the sessions running at devices 135, 140 and 145.

It should be note that provider may also decide not to perform this division, and just send the cost computed for each downstream link (step b)) to all downstream edge devices reached via that downstream link, in the example of Fig. 1 this would mean that devices 140 and 145 would each have to bear session costs of 100.

If the cost division among all edge devices receiving the session is to be performed, the STC mechanism sends a Cost_Adj message to each downstream edge device, with information about the cost of all sessions present in that device.

### Reception of a Cost Leave (Cost_Lve) message from a downstream device:

After receiving such a message (operation 535), the STC mechanism deletes the address of the edge device (operation 540) that sent the Cost_Lve message from the list of edge devices of each session included in the Cost_Lve message.

According to one embodiment in edge access gateways (gateways which are accessed by terminals of individual customers) the STC mechanism does not send Cost_Adj messages and does not process Cost_Lve messages, since the communication with individual customers, reached via this gateway, is done by the DCC mechanism which will be described in more detail in the following.

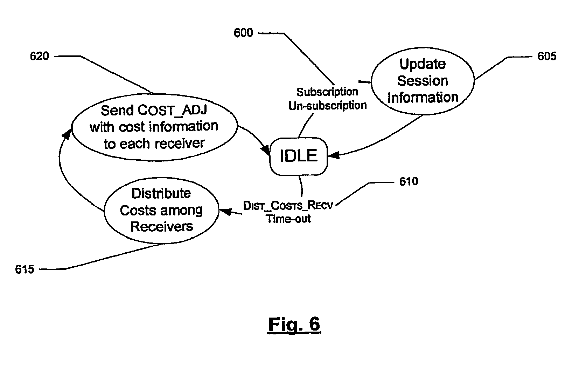

Fig. 6 gives a more detailed description of the operation of the DCC (Distribution of Costs per Customer) mechanism. This mechanism has two triggers, which are the capture of subscription/un-subscription messages from terminals, and the run-out of the timer called Dist_Costs_Recv} timer. After such events the following actions are performed:

### Capture of subscription/un-subscription messages with information about a customer:

The DCC mechanism uses the information collected (operation 600 in Fig. 6) in IGMPv3 and MLDv2 reports or in SIP INVITE/BYE messages, sent by a mechanism like the BRC and used to convey subscribe- or unsubscribe messages, to know which are the channels of each session. After the reception of such messages, the DCC updates (operation 605) the local information of each session, by storing information about new channels, or removing information about existing channels, if the message is to subscribe or un-subscribe a channel, respectively. The updated information will then be used if the updated costs are to be determined. Such an update may e.g. be triggered by a run-out of a timer, as described in the following.

### Run-out of the Dist_Costs_Recv timer:

When this timer runs-out (operation 610 in Fig. 6), the DCC mechanism distributes (operation 615) the cost of each session among the customers that subscribed to it (the cost of each session in the access link was previously computed by a mechanism like the STC). The cost distribution (or its update) is e.g. done using the following steps:
a) Divide the estimated cost of the session in the access link between the channels of the session, based on the ratio between the rate of each channel and the total rate of the session. The rate of each channel, and consequently the rate of the session, may be estimated locally based on meters (if not supplied by the BRC mechanism).
b) For each channel: the DCC equally divides the cost of each channel among the customers using it.
c) For each customer: the DCC adds the per-channel costs estimated in step b), after which the estimate total cost is sent in a Cost_Adj message to the customer's terminal (for instance to a mechanism like BRC).

With the foregoing mechanism the costs of a session from the source to the access point can be distributed among all end users who are running the session on their terminals.

## Claims

1. A method of probing a network to obtain information about the unitary cost of a new session in a multicast network, said method comprising:
transmitting a probe query message containing information about said new session from a downstream device (220) towards an upstream device (235, 230, 225, 210) located in the direction of the source (215) of the session to obtain the unitary cost as the cost per unit of bandwidth for said session;
receiving said probe query message by said upstream device (235, 230, 225, 210), **characterized in that**
if said upstream device is already receiving traffic for said session, estimating the unitary cost of the new session; and
returning a probe answer message back to the source of the probe query message to return information about the unitary cost of the session for the source of the probe query message.

2. The method of claim 1, further comprising:
if said upstream device (230) is not yet receiving traffic for said session, storing information about the channels of the new session in addition to the information about the address of the downstream device, and
forwarding the probe query message further into the direction of the source (215) of the session.

3. The method of claim 1 or 2, further comprising:
estimating said unitary cost based on the total cost for the total available bandwidth in said upstream device (235, 230, 225, 210) and a unitary rate as a fraction of said total available bandwidth, said unitary cost being the transmission cost per unit of bandwidth.

4. The method of one of claims 1 to 3, further comprising:
returning said probe answer message into downstream direction, whereby, in each downstream device through which said probe answer message passes on its way towards the destination of said new session, said unitary cost is updated.

5. The method of claim 4, wherein said updating of said unitary cost comprises:
adding the local unitary upstream cost as received through the probe answer message and the local unitary downstream cost to obtain a total unitary downstream cost.

6. The method of one of claims 1 to 5, further comprising:
receiving a probe answer message containing information about the unitary costs of a session from an upstream device (235, 230, 225, 210),
updating the unitary costs of the session transported by the probe answer message in the device receiving said probe answer message; and
forwarding said probe answer message towards the destination of said session.

7. The method of one of claims 1 to 6, further comprising:
in each upstream device (235, 230, 225, 210) receiving said probe query message, updating session information to store information about said new session, its destination and its channels as well as information about the downstream device from which said probe query message was received.

8. The method of one of claims 1 to 7, further comprising:
if an upstream device (235, 230, 225, 210) receives a probe query message while not receiving traffic for said new session identified by said probe query message, setting a timer, and
if said timer runs out for said new session, erasing the information stored for that session if the rate for that session is still zero.

9. A method of receiver-based controlling the reception data rate of a session which may include multiple channels and wich is been distributed to said receiver over a multicast network, said method comprising:
determining the unitary cost of the session by a method according to one of claims 1 to 8
based on said unitary cost and the rate of each channel as well as based on a budget for transmission costs, determining the number of channels as allowed by the budget, and
running only as many channels for the session as are allowed based on the budget considerations,

10. The method of claim 9, wherein allowable channels are determined based on said budget and further based on the priority of the individual channels.

11. The method of one of claims 9 to 10, further comprising:
in response to an initiation signal to start a session at the destination location, generating a probe query message and forwarding the probe query message towards the source (215) of the session to query about the unitary cost of the session;
receiving an probe answer message from an upstream device (235, 230, 225, 210) located into the direction of the the source of the session and containing information about the unitary cost;
based on the received information, subscribing as many channels as allowed by a budget determined for said session, starting from the channels with highest priority.

12. The method of one of claims 9 to 11, further comprising:
receiving repeatedly a cost adjustment message containing updated information about the costs of the session at the destination of said session;
if said updated costs are lower than the budget for said session, subscribing additional channels as allowed by said budget;
if said updated costs are higher than the budget, unsubscribing channels from said session starting with channels having the lowest priority until the budget criterion is met.

13. The method of one of the preceding claims, wherein
the network is able to handle different classes of service, a session belonging to a certain class and a class having assigned a total value and a corresponding total bandwidth, and wherein for a certain session the unitary cost is estimated dependent on the class to which the session belongs.

14. An apparatus for probing a network to obtain information about the unitary cost of a new session in a multicast network, said apparatus comprising:
a transmitting unit for transmitting a probe query message containing information about said new session from a downstream device (220) towards an upstream device (235, 230, 225, 210) located in the direction of the source (215) of the session to obtain the unitary cost as the cost per unit of bandwidth for said session;
a receiving unit for receiving said probe query message by said upstream device,
**characterized in that** the apparatus comprises a responding unit for, if said apparatus is already receiving traffic for said session, estimating the unitary cost of the new session; and returning a probe answer message back to the source of the probe query message to return information about the unitary cost of the session for the source of the probe query message.

15. The apparatus of claim 14 further comprising:
a storage unit for if said upstream device (235, 230, 225, 210) is not yet receiving traffic for said session, storing information about the channels of the new session in addition to the information about the address of the downstream device, and for
forwarding the probe query message further into the direction of the source (215) of the session.

16. The apparatus of claim 14 or 15, further comprising:
an estimating unit for estimating said unitary cost based on the total cost for the total availble bandwidth in said upstream device and a unitary rate as a fraction of said total available bandwidth, said unitary cost being the transmission cost per unit of bandwidth.

17. The apparatus of one of claims 14 to 16, further comprising:
a returning unit for returning said probe answer message into downstream direction,
and an updating unit for updating said unitary cost in each downstream device through which said probe answer message passes on its way towards the destination of said new session.

18. The apparatus of claim 17, wherein said updating unit comprises:
adding the local unitary upstream cost as received through the probe answer message and the local unitary downstream cost to obtain a total unitary downstream cost.

19. The apparatus of one of claims 14 to 18, further comprising:
a receiving unit or receiving a probe answer message containing information about the unitary costs of a session from an upstream device,
an updating unit for updating the unitary costs of the session transported by the probe answer message in the device receiving said probe answer message; and
a forwarding unit for forwarding said probe answer message towards the destination of said session.

20. The apparatus of one of claims 14 to 19 further comprising:
a unit for in each upstream device (235, 230, 225, 210) receiving said probe query message, updating session information to store information about said new session, its destination and its channels as well as information about the downstream device from which said probe query message was received.

21. The apparatus of one of claims 14 to 20, further comprising:
a timing unit for if an upstream device (235, 230, 225, 210) receives a probe query message while not receiving traffic for said new session identified by said probe query message, setting a timer, and for
if said timer runs out for said new session, erasing the information stored for that session if the rate for that session is still zero.

22. An apparatus for receiver-based controlling the reception data rate of a session which may include multiple channels and wich is been distributed to said receiver over a multicast network, said apparatus comprising:
a first determining unit for determining a unitary cost of a session by a method according to one of claims 1 to 8
a second determining unit for based on the unitary cost and the rate of each channel as well as based on a budget for transmission costs, determining the number of channels as allowed by the budget, and for
running only as many channels for the session as are allowed based on the budget considerations,

23. The apparatus of claim 22 to, further comprising:
a generating unit for in response to an initiation signal to start a session at the destination location, generating a probe query message and forwarding the probe query message towards the source of the session to query about the unitary cost of the session;
a receiving unit for receiving an probe answer message from an upstream device (235, 230, 225, 210) located into the direction of the the source of the session and containing information about the unitary cost;
a subscribing unit for based on the received information, subscribing as many channels as allowed by a budget determined for said session, starting from the channels with highest priority.

24. The apparatus of one of claims 22 to 23, further comprising:
a receiving unit for receiving repeatedly a cost adjustment message containing updated information about the costs of the session at the destination of said session;
the subscribing unit if said updated costs are lower than the budget for said session, subscribing additional channels as allowed by said budget;
the unsubscribing unit if said updated costs are higher than the budget, unsubscribing channels from said session starting with channels having the lowest priority until the budget criterion is met.

25. A computer program comprising computer ecexutable code for when being executed on a computer enabling said computer to carry out a method according to one of claims 1 to 13.

## Patentansprüche

1. Verfahren zum Prüfen eines Netzwerks, um Informationen über die unitären Kosten einer neuen Sitzung in einem Multicast-Netzwerk zu erhalten, wobei das Verfahren umfaßt:
Übertragen einer Prüfanfragenachricht, die Informationen über die neue Sitzung enthält, von einer stromabwärts gelegenen Vorrichtung (220) an eine stromaufwärts gelegene Vorrichtung (235, 230, 225, 210), die in Richtung der Quelle (215) der Sitzung gelegen ist, um die unitären Kosten als Kosten pro Bandbreiteneinheit für die Sitzung zu erhalten;
Empfangen der Prüfanfragenachricht durch die stromaufwärts gelegene Vorrichtung (235, 230, 225, 210), **dadurch gekennzeichnet, dass,**
wenn die stromaufwärts gelegene Vorrichtung bereits Verkehr für die Sitzung empfängt, die unitären Kosten für die neue Sitzung geschätzt werden; und dass eine Prüfantwortnachricht an die Quelle der Prüfanfragenachricht zurückgeschickt wird, um Informationen über die unitären Kosten der Sitzung für die Quelle der Prüfanfragenachricht zurückzusenden.

2. Verfahren nach Anspruch 1, ferner umfassend:
wenn die stromaufwärts gelegene Vorrichtung (230) noch keinen Verkehr für die Sitzung empfängt, Speichern von Informationen über die Kanäle der neuen Sitzung zusätzlich zu den Informationen über die Adresse der stromabwärts gelegenen Vorrichtung, und
Weiterleiten der Prüfanfragenachricht weiter in Richtung der Quelle (215) der Sitzung.

3. Verfahren nach Anspruch 1 oder 2, ferner umfassend:
Schätzen der unitären Kosten basierend auf den Gesamtkosten für die insgesamt verfügbare Bandbreite in der stromaufwärts gelegenen Vorrichtung (235, 230, 225, 210) und einer unitären Rate als Bruchteil der insgesamt verfügbaren Bandbreite, wobei die unitären Kosten die Übertragungskosten pro Bandbreiteneinheit sind.

4. Verfahren nach einem der Ansprüche 1 bis 3, ferner umfassend:
Zurücksenden der Prüfantwortnachricht in die stromabwärts gelegene Richtung, wobei in jeder stromabwärts gelegenen Vorrichtung, durch die die Prüfantwortnachricht auf ihrem Weg zum Bestimmungsort der neuen Sitzung passiert, die unitären Kosten aktualisiert werden.

5. Verfahren nach Anspruch 4, wobei das Aktualisieren der unitären Kosten umfaßt:
Addieren der lokalen unitären Stromaufwärtskosten, wie sie über die Prüfantwortnachricht empfangen wurden, und der lokalen unitären Stromabwärtskosten, um die gesamten unitären Stromabwärtskosten zu erhalten.

6. Verfahren nach einem der Ansprüche 1 bis 5, ferner umfassend:
Empfangen einer Prüfantwortnachricht, die Informationen über die unitären Kosten einer Sitzung von einer stromaufwärts gelegenen Vorrichtung (235, 230, 225, 210) enthält,
Aktualisieren der unitären Kosten der Sitzung, die von der Prüfantwortnachricht transportiert wird, in der Vorrichtung, die die Prüfantwortnachricht empfängt, und
Weiterleiten der Prüfantwortnachricht in Richtung des Bestimmungsorts der Sitzung.

7. Verfahren nach einem der Ansprüche 1 bis 6, ferner umfassend:
Empfangen der Prüfanfragenachricht und Aktualisieren der Sitzungsinformationen in jeder stromaufwärts gelegenen Vorrichtung (235, 230, 225, 210), um Informationen über die neue Sitzung, ihren Bestimmungsort und ihre Kanäle sowie Informationen über die stromabwärts gelegene Vorrichtung zu speichern, von der die Prüfanfragenachricht empfangen wurde.

8. Verfahren nach einem der Ansprüche 1 bis 7, ferner umfassend:
Einstellen eines Zeitgebers, wenn eine stromaufwärts gelegene Vorrichtung (235, 230, 225, 210) eine Prüfanfragenachricht empfängt, während sie keinen Verkehr für die neue Sitzung empfängt, die von der Prüfanfragenachricht identifiziert wurde, und,
wenn der Zeigeber für die neue Sitzung abläuft, Löschen der für diese Sitzung gespeicherten Informationen, wenn die Rate für diese Sitzung immer noch Null beträgt.

9. Verfahren zum empfängerbasierten Steuern der Empfangsdatenrate einer Sitzung, die eine Vielzahl von Kanälen aufweisen kann und die über ein Multicast-Netzwerk an den Empfänger verteilt wird, wobei das Verfahren umfaßt:
Ermitteln der unitären Kosten der Sitzung durch ein Verfahren gemäß einem der Ansprüche 1 bis 8,
Ermitteln der Anzahl von Kanälen, die vom Budget zugelassen wurden, basierend auf den unitären Kosten und der Rate jedes Kanals sowie basierend auf einem Budget für Übertragungskosten, und
Verwenden von lediglich so vielen Kanälen für die Sitzung, wie sie basierend auf den Budgeterwägungen zugelassen wurden.

10. Verfahren nach Anspruch 9, wobei zulässige Kanäle basierend auf dem Budget und ferner basierend auf der Priorität der einzelnen Kanäle ermittelt werden.

11. Verfahren nach einem der Ansprüche 9 bis 10, ferner umfassend:
Erzeugen einer Prüfanfragenachricht und Weiterleiten der Prüfanfragenachricht in Richtung der Quelle (215) der Sitzung in Reaktion auf ein Initüerungssignal zum Starten einer Sitzung am Bestimmungsort, um bezüglich der unitären Kosten der Sitzung anzufragen;
Empfangen einer Prüfantwortnachricht von einer stromaufwärts gelegenen Vorrichtung (235, 230, 225, 210), die in Richtung der Quelle der Sitzung gelegen ist und Informationen über die unitären Kosten enthält;
Abonnieren so vieler Kanäle, wie sie von einem Budget zugelassen wurden, das für die Sitzung ermittelt wurde, beginnend mit den Kanälen mit der höchsten Priorität, basierend auf den empfangenen Informationen.

12. Verfahren nach einem der Ansprüche 9 bis 11, ferner umfassend:
wiederholtes Empfangen einer Kostenanpassungsnachricht, die aktualisierte Informationen über die Kosten der Sitzung enthält, am Bestimmungsort der Sitzung;
Abonnieren zusätzlicher Kanäle, wie sie vom Budget zugelassen wurden, wenn die aktualisierten Kosten niedriger sind als das Budget für die Sitzung;
Abbestellen von Kanälen von der Sitzung, wenn die aktualisierten Kosten höher sind als das Budget, beginnend mit Kanälen, die die niedrigste Priorität aufweisen, bis das Budgetkriterium erfüllt wird.

13. Verfahren nach einem der vorhergehenden Ansprüche, wobei
das Netzwerk in der Lage ist, verschiedene Dienstklassen zu handhaben, wobei eine Sitzung zu einer bestimmten Klasse gehört und einer Klasse ein Gesamtwert und eine entsprechende Gesamtbandbreite zugewiesen wurde, und wobei die unitären Kosten für eine bestimmte Sitzung in Abhängigkeit von der Klasse geschätzt werden, zu der die Sitzung gehört.

14. Vorrichtung zum Prüfen eines Netzwerks, um Informationen über die unitären Kosten einer neuen Sitzung in einem Multicast-Netzwerk zu erhalten, wobei die Vorrichtung umfaßt:
eine Übertragungseinheit zum Übertragen einer Prüfanfragenachricht, die Informationen über die neue Sitzung enthält, von einer stromabwärts gelegenen Vorrichtung (220) an eine stromaufwärts gelegene Vorrichtung (235, 230, 225, 210), die in Richtung der Quelle (215) der Sitzung gelegen ist, um die unitären Kosten als Kosten pro Bandbreiteneinheit für die Sitzung zu erhalten;
eine Empfangseinheit zum Empfangen der Prüfanfragenachricht durch die stromaufwärts gelegene Vorrichtung,
**dadurch gekennzeichnet, dass** die Vorrichtung eine Antworteinheit umfaßt, um, wenn die Vorrichtung bereits Verkehr für die Sitzung empfängt, die unitären Kosten der neuen Sitzung zu schätzen und eine Prüfantwortnachricht an die Quelle der Prüfanfragenachricht zurückzuschicken, um Informationen über die unitären Kosten der Sitzung für die Quelle der Prüfanfragenachricht zurückzusenden.

15. Vorrichtung nach Anspruch 14, ferner umfassend:
eine Speichereinheit zum Speichern von Informationen über die Kanäle der neuen Sitzung zusätzlich zu den Informationen über die Adresse der stromabwärts gelegenen Vorrichtung, wenn die stromaufwärts gelegene Vorrichtung (235, 230, 225, 210) noch keinen Verkehr für die Sitzung empfängt, und zum
Weiterleiten der Prüfanfragenachricht weiter in Richtung der Quelle (215) der Sitzung.

16. Vorrichtung nach Anspruch 14 oder 15, ferner umfassend:
eine Schätzeinheit zum Schätzen der unitären Kosten basierend auf den Gesamtkosten für die insgesamt verfügbare Bandbreite in der stromaufwärts gelegenen Vorrichtung und einer unitären Rate als Bruchteil der insgesamt verfügbaren Bandbreite, wobei die unitären Kosten die Übertragungskosten pro Bandbreiteneinheit sind.

17. Vorrichtung nach einem der Ansprüche 14 bis 16, ferner umfassend:
eine Rücksendeeinheit zum Zurücksenden der Prüfantwortnachricht in die stromabwärts gelegene Richtung,
und eine Aktualisierungseinheit zum Aktualisieren der unitären Kosten in jeder stromabwärts gelegenen Vorrichtung, durch die die Prüfantwortnachricht auf ihrem Weg in Richtung des Bestimmungsortes der neuen Sitzung passiert.

18. Vorrichtung nach Anspruch 17, wobei die Aktualisierungseinheit umfaßt:
Addieren der lokalen unitären Stromaufwärtskosten, wie sie über die Prüfantwortnachricht empfangen wurden, und der lokalen unitären Stromabwärtskosten, um die gesamten unitären Stromabwärtskosten zu erhalten.

19. Vorrichtung nach einem der Ansprüche 14 bis 18, ferner umfassend:
eine Empfangseinheit zum Empfangen einer Prüfantwortnachricht, die Informationen über die unitären Kosten einer Sitzung von einer stromaufwärts gelegenen Vorrichtung enthält,
eine Aktualisierungseinheit zum Aktualisieren der unitären Kosten der Sitzung, die von der Prüfantwortnachricht transportiert wird, in der Vorrichtung, die die Prüfantwortnachricht empfängt; und
eine Weiterleitungseinheit zum Weiterleiten der Prüfantwortnachricht in Richtung des Bestimmungsorts der Sitzung.

20. Vorrichtung nach einem der Ansprüche 14 bis 19, ferner umfassend:
eine Einheit, um in jeder stromaufwärts gelegenen Vorrichtung (235, 230, 225, 210) die Prüfanfragenachricht zu empfangen und die Sitzungsinformationen zu aktualisieren, um Informationen über die neue Sitzung, ihren Bestimmungsort und ihre Kanäle sowie Informationen über die stromabwärts gelegene Vorrichtung zu speichern, von der die Prüfanfragenachricht empfangen wurde.

21. Vorrichtung nach einem der Ansprüche 14 bis 20, ferner umfassend:
eine Zeitgebereinheit, um einen Zeitgeber einzustellen, wenn eine stromaufwärts gelegene Vorrichtung (235, 230, 225, 210) eine Prüfanfragenachricht empfängt, während sie keinen Verkehr für die neue Sitzung empfängt, die von der Prüfanfragenachricht identifiziert wurde, und um,
wenn der Zeigeber für die neue Sitzung abläuft, die für diese Sitzung gespeicherten Informationen zu löschen, wenn die Rate für diese Sitzung immer noch Null beträgt.

22. Vorrichtung zum empfängerbasierten Steuern der Empfangsdatenrate einer Sitzung, die eine Vielzahl von Kanälen aufweisen kann und die über ein Multicast-Netzwerk an den Empfänger verteilt wird, wobei die Vorrichtung umfaßt:
eine erste Ermittlungseinheit zum Ermitteln von unitären Kosten einer Sitzung durch ein Verfahren nach einem der Ansprüche 1 bis 8,
eine zweite Ermittlungseinheit, um basierend auf den unitären Kosten und der Rate jedes Kanals sowie basierend auf einem Budget für Übertragungskosten die Anzahl von Kanälen zu ermitteln, wie sie vom Budget zugelassen wurden, und um
nur so viele Kanäle für die Sitzung zu verwenden, wie sie basierend auf den Budgeterwägungen zugelassen wurden.

23. Vorrichtung nach Anspruch 22, ferner umfassend:
eine Erzeugungseinheit, um in Reaktion auf ein Initüerungssignal zum Starten einer Sitzung am Bestimmungsort eine Prüfanfragenachricht zu erzeugen und die Prüfanfragenachricht in Richtung der Quelle der Sitzung weiterzuleiten, um bezüglich der unitären Kosten der Sitzung anzufragen;
eine Empfangseinheit zum Empfangen einer Prüfantwortnachricht von einer stromaufwärts gelegenen Vorrichtung (235, 230, 225, 210), die in Richtung der Quelle der Sitzung gelegen ist und Informationen über die unitären Kosten enthält;
eine Abonniereinheit zum Abonnieren so vieler Kanäle, wie sie von einem Budget zugelassen wurden, das für die Sitzung ermittelt wurde, beginnend mit den Kanälen mit der höchsten Priorität, basierend auf den empfangenen Informationen.

24. Vorrichtung nach einem der Ansprüche 22 bis 23, ferner umfassend:
eine Empfangseinheit zum wiederholten Empfangen einer Kostenanpassungsnachricht, die aktualisierte Informationen über die Kosten der Sitzung enthält, am Bestimmungsort der Sitzung;
eine Abonniereinheit zum Abonnieren zusätzlicher Kanäle, wie sie vom Budget zugelassen wurden, wenn die aktualisierten Kosten niedriger sind als das Budget für die Sitzung;
eine Abbestelleinheit zum Abbestellen von Kanälen von der Sitzung, beginnend mit Kanälen, die die niedrigste Priorität aufweisen, bis das Budgetkriterium erfüllt wird, wenn die aktualisierten Kosten höher sind als das Budget.

25. Computerprogramm, der von einem Computer ausführbaren Code umfaßt, um, wenn er auf einem Computer ausgeführt wird, es dem Computer zu ermöglichen, ein Verfahren nach einem der Ansprüche 1 bis 13 auszuführen.

## Revendications

1. Procédé permettant de sonder un réseau afin d'obtenir des informations concernant le coût unitaire d'une nouvelle session dans un réseau multidiffusion, ledit procédé comprenant :
la transmission d'un message de demande de sonde contenant des informations concernant ladite nouvelle session depuis un dispositif en aval (220) vers un dispositif en amont (235, 230, 225, 210) localisé dans la direction de la source (215) de la session afin d'obtenir le coût unitaire comme le coût par unité de bande passante pour ladite session ;
la réception dudit message de demande de sonde par ledit dispositif en amont (235, 230, 225, 210), **caractérisé en ce que**
si ledit dispositif en amont reçoit déjà du trafic pour ladite session, estimer le coût unitaire de la nouvelle session ; et renvoyer un message de réponse de sonde à la source du message de demande de sonde afin de retourner des informations concernant le coût unitaire de la session pour la source du message de demande de sonde.

2. Procédé selon la revendication 1, comprenant en outre ;
si ledit dispositif en amont (230) ne reçoit pas encore de trafic pour ladite session, stocker des informations concernant les canaux de la nouvelle session en plus des informations concernant l'adresse du dispositif en aval, et
transférer le message de demande de sonde en outre dans la direction de la source (215) de la session.

3. Procédé selon la revendication 1 ou 2, comprenant en outre :
estimer ledit coût unitaire sur la base du coût total pour la bande passante totale disponible dans ledit dispositif en amont (235, 230, 225, 210) et un débit unitaire comme une fraction de ladite bande passante totale disponible, ledit coût unitaire étant le coût de transmission par unité de bande passante.

4. Procédé selon l'une des revendications 1 à 3, comprenant en outre :
renvoyer ledit message de réponse de sonde dans la direction aval, moyennant quoi dans chaque dispositif en aval à travers lequel ledit message de réponse de sonde passe en direction de la destination de ladite nouvelle session, ledit coût unitaire est mis à jour.

5. Procédé selon la revendication 4, dans lequel ladite mise à jour dudit coût unitaire comprend :
additionner le coût unitaire local amont tel que reçu par l'intermédiaire du message de réponse de sonde et du coût unitaire local aval afin d'obtenir un coût unitaire total aval.

6. Procédé selon l'une des revendications 1 à 5, comprenant en outre :
recevoir un message de réponse de sonde contenant des informations concernant les coûts unitaires d'une session en provenance d'un dispositif en amont (235, 230, 225, 210),
mettre à jour les coûts unitaires de la session transportés par le message de réponse de sonde dans le dispositif recevant ledit message de réponse de sonde ; et
transférer ledit message de réponse de sonde vers la destination de ladite session.

7. Procédé selon l'une des revendications 1 à 6, comprenant en outre :
dans chaque dispositif en amont (235, 230, 225, 210) recevant ledit message de demande de sonde, mettre à jour l'informations de session pour stocker des informations concernant ladite nouvelle session, sa destination et ses canaux ainsi que des informations concernant le dispositif en aval duquel ledit message de demande de sonde a été reçu.

8. Procédé selon l'une des revendications 1 à 7, comprenant en outre :
si un dispositif en amont (235, 230, 225, 210) reçoit un message de demande de sonde alors qu'il ne reçoit pas de trafic pour ladite nouvelle session identifiée par ledit message de demande de sonde, établir un temporisateur, et
si ledit temporisateur arrive à expiration pour ladite nouvelle session, effacer les informations stockées pour cette session si le débit pour cette session est toujours zéro.

9. Procédé de commande basée sur récepteur du débit de données de réception d'une session qui peut inclure de multiples canaux et qui est distribuée audit récepteur sur un réseau multidiffusion, ledit procédé comprenant :
déterminer le coût unitaire de la session par un procédé selon l'une des revendications 1 à 8, et sur la base dudit coût unitaire et du débit de chaque canal ainsi que sur la base d'un budget pour les coûts de transmission, déterminer le nombre de canaux tels qu'autorisés par le budget, et
exécuter uniquement le nombre de canaux maximum pour la session tels qu'autorisés sur la base des considérations de budget.

10. Procédé selon la revendication 9, dans lequel les canaux admissibles sont déterminés sur la base dudit budget et en outre sur la base de la priorité de canaux individuels.

11. Procédé selon l'une des revendications 9 à 10, comprenant en outre :
en réponse à un signal d'initiation pour démarrer une session au niveau de la localisation de destination, générer un message de demande de sonde et transférer le message de demande de sonde vers la source (215) de la session pour demander le coût unitaire de la session ;
recevoir un message de réponse de sonde en provenance d'un dispositif en amont (235, 230, 225, 210) localisé dans la direction de la source de la session et contenant des informations concernant le coût unitaire ;
sur la base des informations reçues, s'abonner à autant de canaux qu'autorisés par un budget déterminé pour ladite session, en commençant par les canaux avec la priorité la plus élevée.

12. Procédé selon l'une des revendications 9 à 11, comprenant en outre ;
la réception répétée d'un message d'ajustement de coût contenant des informations mises à jour concernant les coûts de la session au niveau de la destination de ladite session ;
si lesdits coûts mis à jour sont inférieurs au budget pour ladite session, s'abonner à des canaux additionnels tels qu'autorisés par ledit budget ;
si lesdits coûts mis à jour sont supérieurs au budget, se désabonner des canaux de ladite session en commençant par les canaux ayant la priorité la plus basse jusqu'à ce que le critère de budget soit satisfait.

13. Procédé selon l'une des revendications précédentes, dans lequel
le réseau est capable de prendre en charge différentes classes de service, une session appartenant à une certaine classe et une classe se voyant assigner une valeur totale et une bande passante totale correspondante, et dans lequel pour une certaine session, le coût unitaire est estimé en fonction de la classe à laquelle appartient la session.

14. Appareil destiné à sonder un réseau afin d'obtenir des informations concernant le coût unitaire d'une nouvelle session dans un réseau multidiffusion, ledit appareil comprenant :
une unité de transmission pour transmettre un message de demande de sonde contenant des informations concernant ladite nouvelle session depuis un dispositif en aval (220) vers un dispositif en amont (235, 230, 225, 210) localisé dans la direction de la source (215) de la session afin d'obtenir le coût unitaire comme le coût par unité de bande passante pour ladite session ;
une unité de réception pour recevoir ledit message de demande de sonde par ledit dispositif en amont,
**caractérisé en ce que** l'appareil comprend une unité de réponse pour, si ledit appareil reçoit déjà du trafic pour ladite session, estimer le coût unitaire de la nouvelle session ; et renvoyer un message de réponse de sonde à la source du message de demande de sonde afin de retourner des informations concernant le coût unitaire de la session pour la source du message de demande de sonde.

15. Appareil selon la revendication 14, comprenant en outre :
une unité de stockage pour, si ledit dispositif en amont (235, 230, 225, 210) ne reçoit pas encore de trafic pour ladite session, stocker des informations concernant les canaux de la nouvelle session en plus des informations concernant l'adresse du dispositif en aval, et pour
transférer le message de demande de sonde en outre dans la direction de la source (215) de la session.

16. Appareil selon la revendication 14 ou 15, comprenant en outre :
une unité d'estimation pour estimer ledit coût unitaire sur la base du coût total pour la bande passante totale disponible dans ledit dispositif en amont et un débit unitaire comme une fraction de ladite bande passante totale disponible, ledit coût unitaire étant le coût de transmission par unité de bande passante.

17. Appareil selon l'une des revendications 14 à 16, comprenant en outre :
une unité de renvoi pour renvoyer ledit message de réponse de sonde dans la direction aval,
et une unité de mise à jour pour mettre à jour ledit coût unitaire dans chaque dispositif en aval à travers lequel ledit message de réponse de sonde passe en direction de la destination de ladite nouvelle session.

18. Appareil selon la revendication 17, dans lequel ladite unité de mise à jour comprend :
l'addition du coût unitaire local amont tel que reçu par l'intermédiaire du message de réponse de sonde et du coût unitaire local aval afin d'obtenir un coût unitaire total aval.

19. Appareil selon l'une des revendications 14 à 18, comprenant en outre :
une unité de réception pour recevoir un message de réponse de sonde contenant des informations concernant les coûts unitaires d'une session en provenance d'un dispositif en amont,
une unité de mise à jour pour mettre à jour les coûts unitaires de la session transportés par le message de réponse de sonde dans le dispositif recevant ledit message de réponse de sonde ; et
une unité de transfert pour transférer ledit message de réponse de sonde vers la destination de ladite session.

20. Appareil selon l'une des revendications 14 à 19, comprenant en outre :
une unité pour, dans chaque dispositif en amont (235, 230, 225, 210) recevant ledit message de demande de sonde, mettre à jour des informations de session afin de stocker des informations concernant ladite nouvelle session, sa destination et ses canaux ainsi que des informations concernant le dispositif en aval duquel ledit message de demande de sonde a été reçu.

21. Appareil selon l'une des revendications 14 à 20, comprenant en outre :
une unité de temporisation pour, si un dispositif en amont (235, 230, 225, 210) reçoit un message de demande de sonde alors qu'il ne reçoit pas de trafic pour ladite nouvelle session identifiée par ledit message de demande de sonde, établir un temporisateur, et pour
si ledit temporisateur arrive à expiration pour ladite nouvelle session, effacer les informations stockées pour cette session si le débit pour cette session est toujours zéro.

22. Appareil de commande basée sur récepteur du débit de données de réception d'une session qui peut inclure de multiples canaux et qui est distribuée audit récepteur sur un réseau multidiffusion, ledit appareil comprenant :
une première unité de détermination pour déterminer un coût unitaire d'une session par un procédé selon l'une des revendications 1 à 8 et une seconde unité de détermination pour, sur la base du coût unitaire et du débit de chaque canal ainsi que sur la base d'un budget pour les coûts de transmission, déterminer le nombre de canaux tels qu'autorisés par le budget, et pour
exécuter uniquement le nombre de canaux maximum pour la session tels qu'autorisés sur la base des considérations de budget.

23. Appareil selon la revendication 22, comprenant en outre :
une unité de génération pour, en réponse à un signal d'initiation pour démarrer une session au niveau de la localisation de destination, générer un message de demande de sonde et transférer le message de demande de sonde vers la source de la session pour demander le coût unitaire de la session ;
une unité de réception pour recevoir un message de réponse de sonde en provenance d'un dispositif en amont (235, 230, 225, 210) localisé dans la direction de la source de la session et contenant des informations concernant le coût unitaire ;
une unité d'abonnement pour, sur la base des informations reçues, s'abonner à autant de canaux qu'autorisés par un budget déterminé pour ladite session, en commençant par les canaux avec la priorité la plus élevée.

24. Appareil selon l'une des revendications 22 à 23, comprenant en outre :
une unité de réception pour recevoir de façon répétée un message d'ajustement de coût contenant des informations mises à jour concernant les coûts de la session au niveau de la destination de ladite session ;
l'unité d'abonnement, si lesdits coûts mis à jour sont inférieurs au budget pour ladite session, s'abonnant à des canaux additionnels tels qu'autorisés par ledit budget ;
l'unité de désabonnement, si lesdits coûts mis à jour sont supérieurs au budget, se désabonnant des canaux de ladite session en commençant par les canaux ayant la priorité la plus basse jusqu'à ce que le critère de budget soit satisfait.

25. Programme informatique comprenant un code exécutable par ordinateur pour, lorsqu'il est exécuté sur un ordinateur, permettre que ledit ordinateur effectue un procédé selon l'une des revendications 1 à 13.
